# EUROPEAN PATENT APPLICATION

(11) **EP 4 748 922 A1**
(43) Date of publication of application: **27.05.2026**
(21) Application number: 24843173.6
(22) Date of filing: 18.07.2024
(51) Int. Cl.: C12N 1/20, A23L 33/135, A61K 35/747, A61P 19/00, A61P 19/08, A61P 29/00, A61P 31/12, A61P 35/00, A61P 37/02, A61P 37/04

(54) **NOVEL LACTIC ACID BACTERIA, NOVEL LACTIC ACID BACTERIA-CONTAINING COMPOSITION, AND METHOD FOR PRODUCING SAME**

(30) Priority: 18.07.2023 JP 2023117005
(71) Applicant: Morinaga Milk Industry Co., Ltd., Minato-ku Tokyo 105-7122 (JP)
(72) Inventor: ISHIHARA, Sayaka, Zama-shi, Kanagawa 252-8583 (JP); NISHIMURA, Tatsuki, Zama-shi, Kanagawa 252-8583 (JP); SEN, Akira, Zama-shi, Kanagawa 252-8583 (JP)
(74) Representative: Hoffmann Eitle
(86) International application number: PCT/JP2024/025763
(87) International publication number: WO 2025/018384

(57) **Abstract**

A problem of the invention is to provide a bacterium belonging to *Lactobacillus helveticus* having an excellent IFN production-inducing activity. A bacterium classified as *Lactobacillus helveticus* which can activate plasmacytoid dendritic cells (pDCs) and/or myeloid dendritic cells (mDCs) and induce production of type I IFN and which induces the production of type I IFN in an amount 1.5 times or more higher than that of *Lactobacillus helveticus* SBT2171 (accession number: Ferm BP-5445).

## Description

### Technical Field

The present invention relates to a novel bacterium belonging to *Lactobacillus helveticus,* a composition containing a novel bacterium or a culture or processed bacterial cells of the novel bacterium and a production method thereof.

### Background Art

Immunity is a biological defense mechanism inherent in many animals and plays a role of eliminating pathogens which enter from outside, such as bacteria and viruses, and tumor cells generated in the body. There are two immune systems, namely the innate immune system and the acquired immune system, which have biological defense functions. The innate immune system is mainly responsible for the primary response in bacterial or viral infection, and in particular, dendritic cells are potent and important constitutive cells.

Dendritic cells are highly plastic cells and include many subspecies, and as dendritic cells which induce type I interferon (type I IFN) exhibiting inhibitory activity on viral proliferation through activation of a Toll-like receptor (TLR), plasmacytoid dendritic cells (pDCs) and myeloid dendritic cells (mDCs) are known.

pDCs are main cells producing type I IFNs in the body. In order that pDCs induce type I IFN production, stimulation of TLRs in endosomes such as TLR7/TLR9, of TLRs, is required.

mDCs induce the production of type I IFNs through activation of TLR3 and the like. Moreover, through bacterial infection, mDCs release inflammatory cytokines, mainly interleukin-12 (IL-12), tumor necrosis factor-a (TNF-α) and the like, and induce activation of helper T cells (CD4+ T cells).

Here, according to recent studies, it has been found that there are lactic acid bacteria which exhibit innate immunity-activating activity.

Patent Literature 1 discloses that *Lactobacillus lactis* (*Lactococcus Lactis*) activates pDCs and induces IFN production.

Patent Literature 2 discloses that *Lactobacillus helveticus* SBT2171 (FERM BP-5445) has an activity of increasing pDCs.

### Citation List

### Patent Literature

Patent Literature 1: JP6170190B
Patent Literature 2: JP6705628B

### Summary of Invention

### Technical Problem

As disclosed in Patent Literature 1, it is known that *Lactobacillus lactis* has an IFN production-inducing activity, but these days, with raised awareness of prevention of viral infection such as COVID-19, a bacterium which exhibits a superior IFN production-inducing activity is desired.

Moreover, SBT2171 described in Patent Literature 2 is a bacterial strain selected using the activity of promoting the proliferation of pDCs as an indicator, but not the IFN production-inducing activity. The examination on *Lactobacillus helveticus* has been insufficient in terms of the IFN production-inducing activity.

In view of the circumstances, a problem of the invention is to provide a bacterium belonging to *Lactobacillus helveticus* having an excellent IFN production-inducing activity.

### Solution to Problem

After intensive studies and efforts, the present inventors have found a bacterial strain of *Lactobacillus helveticus* which exhibits a superior type I IFN production-inducing activity to those of *Lactobacillus lactis* JCM5805, which is specifically described in Patent Literature 1, and *Lactobacillus helveticus* SBT2171, which is described in Patent Literature 2, and the invention has been thus completed. The invention which solves the problem is as follows.
[1] A bacterium classified as *Lactobacillus helveticus* which can activate plasmacytoid dendritic cells (pDCs) and/or myeloid dendritic cells (mDCs) and induce production of type I IFN and which induces the production of type I IFN in an amount 1.5 times or more higher than that of *Lactobacillus helveticus* SBT2171 (accession number: Ferm BP-5445).
[2] *Lactobacillus helveticus* MCC2430 (accession number: NITE BP-03882).
[3] A composition containing at least one kind selected from the bacterium according to [1] or [2], a culture of the bacterium and processed bacterial cells of the bacterium.
[4] The composition according to [3] which is a composition for inducing production of type I IFN.
[5] The composition according to [3] which is at least one kind selected from the group consisting of an antiviral composition, an immunostimulating composition, a composition for treating or preventing an immune disease, an anticancer composition, an anti-inflammatory composition and a composition for preventing a decrease in bone strength.
[6] The composition according to any one of [3] to [5] which is a food or a drink or a pharmaceutical product.
[7] A method for producing a composition including a step of adding at least one kind selected from the bacterium according to [1] or [2], a culture of the bacterium and processed bacterial cells of the bacterium to a raw material.
[8] The production method according to [7] including a step of fermenting the raw material with the bacterium, in which the composition is a fermented composition.

### Advantageous Effects of Invention

The bacterium of the invention potently induces type I IFN production by activation of pDCs and/or mDCs.

The composition of the invention induces the production of type I IFN in a subject which has taken the composition and exhibits an antiviral effect, an immunostimulating effect, an effect of treating or preventing an immune disease, an anticancer effect, an anti-inflammatory effect, an effect of preventing a decrease in bone strength or the like.

According to the production method of the invention, a composition which exhibits the above effects can be produced.

### Brief Description of Drawings

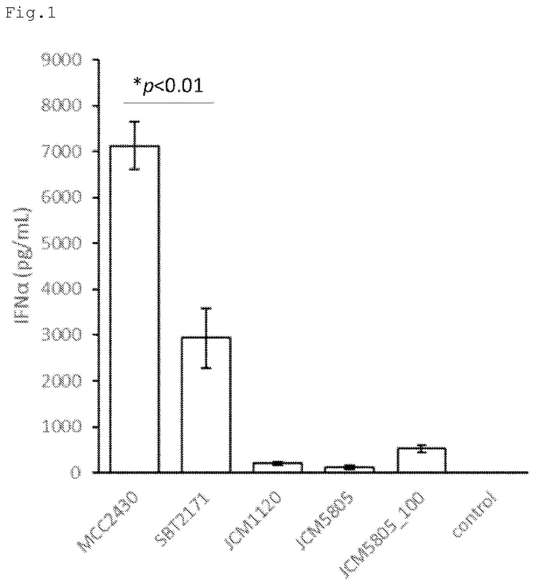

[FIG. 1] Fig. 1 is a bar chart showing the concentrations of IFNα in the culture supernatants measured by flow cytometry using an IFNα assay kit. The test was conducted three times, and the average values are shown in the graph. The error bars in the graph show standard deviations. The p values in the figure were obtained by Student's t-test. Here, sterilized bacterial cells of the bacterial strains were added in an amount 10 times higher than that of the dendritic cells in terms of the cell count ratio, but for JCM5805, a test in which the sterilized bacterial cells in 100 times the amount were added was conducted additionally. In the figure, "JCM5805_100" shows the test results of JCM5805 in which the sterilized bacterial cells were added in an amount 100 times higher than that of the dendritic cells.

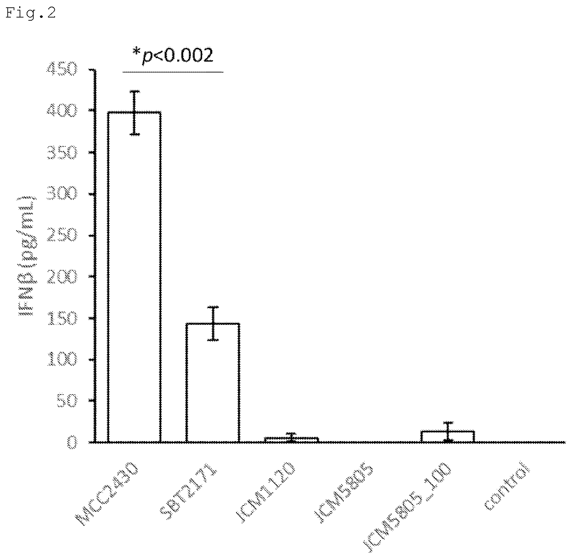

[FIG. 2] Fig. 2 is a bar chart showing the concentrations of IFNβ in the culture supernatants measured by ELISA using an IFNβ assay kit. The test was conducted three times, and the average values are shown in the graph. The error bars in the graph show standard deviations. The p values in the figure were obtained by Student's t-test. Here, sterilized bacterial cells of the bacterial strains were added in an amount 10 times higher than that of the dendritic cells in terms of the cell count ratio, but for JCM5805, a test in which the sterilized bacterial cells in 100 times the amount were added was conducted additionally. In the figure, "JCM5805_100" shows the test results of JCM5805 in which the sterilized bacterial cells were added in an amount 100 times higher than that of the dendritic cells.

### Description of Embodiments

Preferable embodiments of the invention will be explained. However, the invention is not limited to the following preferable embodiments and can be changed freely within the scope of the invention. In the present specification, the percentages are based on mass unless otherwise specified.

### <Bacterium Which Can Induce Type I IFN Production>

The invention relates to a bacterium classified as *Lactobacillus helveticus* which can activate plasmacytoid dendritic cells (pDCs) and/or myeloid dendritic cells (mDCs) and induce type I IFN production.

More specifically, the bacterium of the invention is a bacterium classified as *Lactobacillus helveticus* which induces the production of type I IFN in an amount 1.5 times or more higher than that of *Lactobacillus helveticus* SBT2171 (accession number: Ferm BP-5445).

A bacterium classified as *Lactobacillus helveticus* with which the production amount of type I IFN induced through activation of pDCs and/or mDCs is 1.5 times or more higher than that of SBT2171 is included in the scope of the invention. The conditions of the test for measuring the production amount of type I IFN to examine whether the bacterium belongs to the invention is not particularly restricted. In the present specification, the test for examining whether the bacterium belongs to the invention is called "confirmation test" for the convenience of explanation.

An example of the confirmation test is a testing system of adding bacterial cells to a mixture culture system culturing pDCs and/or mDCs and measuring the concentration of type I IFN in the culture supernatant. When the concentration of type I IFN measured is 1.5 times or more higher than that of the case in which bacterial cells of SBT2171 are added under the same condition, it can be determined that the bacterium as the subject of the confirmation test belongs to the scope of the invention.

The culture system to which the bacterial cells are added may be a culture system culturing either pDCs or mDCs alone or a mixed culture system of pDCs and mDCs.

The scope of the invention includes all of an aspect which activates pDCs and induces the production of type I IFN but does not activate mDCs, an aspect which activates mDCs and induces the production of type I IFN but does not activate pDCs and an aspect which activates both pDCs and mDCs and induces the production of type I IFN.

The bacterial cells added to the culture system of pDCs and/or mDCs may be viable cells or dead cells. When dead cells are used, heat sterilized cells are preferably used.

The number of the bacterial cell added to the culture system of pDCs and/or mDCs is not limited. For example, the bacterial cells at a count which is 10 to 100 times the number of cells of pDCs and/or mDCs can be added. Specifically, an aspect of adding the bacterial cells at a count which is 10 times the number of the cells of pDCs and/or mDCs to a culture system thereof is preferably exemplified.

The origin tissue of the pDCs and/or the mDCs used for the confirmation test is not limited. For example, those derived from peripheral blood mononuclear cells may be used, or those derived from bone marrow cells may also be used.

The origin animal species is not limited, and examples thereof include mammals such as human, monkey, mouse, rat, rabbit, dog and cat. Preferably, human-derived pDCs and/or mDCs are used.

The method for measuring the concentration of type I IFN in the culture supernatant of pDCs and/or mDCs is not particularly limited, and flow cytometry or ELISA can be used.

A commercial kit can also be used for the measurement. Examples thereof include an IFNα assay kit (Cytometric Bead Array Kit, Becton Dickinson) and an IFNβ assay kit (Human IFN-beta DuoSet, manufactured by R&D systems).

Type I IFNs of mammals are composed of different classes of eight kinds or more including IFNα, IFNβ, IFNδ, IFNε, IFNκ, IFNω, IFNτ and IFNζ. In human, IFNα, IFNβ, IFNε, IFNκ and IFNω have been found. The scope of the invention also includes an aspect inducing the production of any interferon corresponding to type I IFNs.

In a preferable aspect of the invention, the production of IFNα and/or IFNβ is induced.

The scope of the invention includes all of an aspect which induces the production of IFNα but does not induce the production of IFNβ, an aspect which induces the production of IFNβ but does not induce the production of IFNα and an aspect which induces the production of both IFNα and IFNβ.

In a preferable aspect of the invention, the production of both IFNα and IFNβ is induced.

Type I IFN measured in the confirmation test may be any interferon corresponding to type I IFNs, and is preferably IFNα or IFNβ. In this case, the subject of measurement may be either IFNα or IFNβ or both IFNα and IFNβ.

The period between the addition of the bacterial cells to the culture system of pDCs and/or mDCs and the measurement of the concentration of type I IFN in the culture supernatant is preferably 12 to 48 hours, preferably 20 to 28 hours, further preferably 24 hours.

The confirmation test may be conducted once or multiple times.

When the confirmation test is conducted multiple times, the average value of the measured values of the concentration of type I IFN obtained in the confirmation tests may be compared with that of SBT2171. In this case, the number of the tests is preferably three or more.

As described above, the bacterium of the invention induces the production of type I IFN in an amount 1.5 times or more higher than that of SBT2171. In a preferable embodiment, the bacterium induces the production of type I IFN in an amount 1.8 times or more, more preferably two times or more, further preferably 2.2 times or more higher than that of SBT2171.

In a preferable embodiment, the bacterium induces the production of IFNα and/or IFNβ in an amount 1.5 times or more higher than that of SBT2171. In a preferable embodiment, the bacterium induces the production of IFNα and/or IFNβ in an amount 1.8 times or more, more preferably two times or more, further preferably 2.2 times or more higher than that of SBT2171.

In a specific embodiment, the bacterium induces the production of IFNα in an amount 1.5 times or more, preferably 1.8 times or more, more preferably two times or more, further preferably 2.2 times or more, further preferably 2.3 times or more higher than that of SBT2171.

In another specific embodiment, the bacterium induces the production of IFNβ in an amount 1.5 times or more, preferably 1.8 times or more, more preferably two times or more, further preferably 2.2 times or more, further preferably 2.4 times or more, further preferably 2.6 times or more, further preferably 2.7 times or more higher than that of SBT2171.

In a further specific embodiment, the bacterium induces the production of IFNα in an amount 1.5 times or more, preferably 1.8 times or more, more preferably two times or more, further preferably 2.2 times or more, further preferably 2.3 times or more higher than that of SBT2171, and
induces the production of IFNβ in an amount 1.5 times or more, preferably 1.8 times or more, more preferably two times or more, further preferably 2.2 times or more, further preferably 2.4 times or more, further preferably 2.6 times or more, further preferably 2.7 times or more higher.

The bacterium of the invention may be in the form of a bacterial strain which has been isolated and cloned as a genetically homogeneous cell population or the form of an uncloned genetically nonhomogeneous cell population.

The form of a genetically nonhomogeneous cell population is of course included in the scope of the invention as long as the cell population exhibits the activity of inducing the production of type I IFN explained above as a characteristic of the whole population.

Of the bacteria of the invention, a bacterial strain which has been isolated and cloned as a genetically homogeneous cell population is *Lactobacillus helveticus* MCC2430 (accession number: NITE BP-03882). The bacterial strain will be described below.

The bacterium of the invention can be proliferated easily by culturing the bacterium. The culture method is not particularly limited as long as *Lactobacillus helveticus* can be proliferated, and a method which is generally used for culturing a lactic acid bacterium can be used with appropriate modification as necessary. For example, the culture temperature may be 25 to 50°C and is preferably 35 to 42°C. The bacterium is preferably cultured under an anaerobic condition and can be cultured, for example, while sending an anaerobic gas such as carbon dioxide gas. Furthermore, the bacterium may be cultured under a microaerophilic condition such as static liquid culture.

The culture medium for culturing the bacterium of the invention is not particularly limited, and a culture medium which is generally used for culturing a lactic acid bacterium can be used with appropriate modification as necessary. That is, as a carbon source, for example, saccharides such as galactose, glucose, fructose, mannose, cellobiose, maltose, lactose, sucrose, trehalose, starch, starch hydrolysate and molasses can be used depending on the assimilation properties. As a nitrogen source, for example, ammonia and ammonium salts and nitrates such as ammonium sulfate, ammonium chloride and ammonium nitrate can be used. Moreover, as an inorganic salt, for example, sodium chloride, potassium chloride, potassium phosphate, magnesium sulfate, calcium chloride, calcium nitrate, manganese chloride, and ferrous sulfate can be used. Furthermore, organic components such as peptone, soybean powder, a defatted soybean cake, meat extract and yeast extract may also be used. As a modified culture medium, for example, MRS culture medium can be preferably used.

The bacterium of the invention can be provided as a biologically purely cultured culture.

### <Lactobacillus helveticus MCC2430>

The invention also relates to *Lactobacillus helveticus* MCC2430 (accession number: NITE BP-03882). The strain is simply called MCC2430 below.

MCC2430 of the invention was deposited, as a novel bacterial strain due to the bacteriological properties and features below, on April 13, 2023 to NITE Patent Microorganisms Depositary, National Institute of Technology and Evaluation (NPMD) (address: # 122, 2-5-8 Kazusakamatari, Kisarazu-shi, Chiba 292-0818, Japan) as *Lactobacillus helveticus* (accession number: NITE BP-03882). The bacterial strain can be generally acquired from the above preservation organization.

MCC2430 of the invention is not limited to the deposited bacterial strain and may be a substantially equivalent bacterial strain of the deposited bacterial strain.

The substantially equivalent bacterial strain may be a bacterial strain corresponding to at least one of (1) to (3) below.
(1) A bacterial strain which is identified as the identical bacterial strain by the Randomly Amplified Polymorphic DNA (RAPD) method or the Pulsed-field gel electrophoresis (PFGE) method (described in FAO/WHO (2006) Probiotics in Food: Health and Nutritional Properties and Guidelines for Evaluation. Report of a Joint FAO/WHO Expert Consultation on Evaluation of Health and Nutritional Properties of Probiotics in Food Including Powder Milk with Live Lactic Acid Bacteria, Cordoba, Argentina, 1-4 October 2001 [and] Report of a Joint FAO/WHO Working Group on Drafting Guidelines for the Evaluation of Probiotics in Food, London, Ontario, Canada, 30 April-1 May 2002. FAO Food and Nutrition Paper 85, Food and Agriculture Organization of the United Nations, World Health Organization, Rome. Page 43)
(2) A bacterial strain which possesses the deposited bacterial strain-derived genes only and does not have any exogenous genes and which has a DNA identity of 95% or more
(3) A bacterial strain bred from the bacterial strain or a bacterial strain which has undergone genetic engineering modification, mutation or spontaneous mutation, where the bacterial strain has an equivalent type I IFN production-inducing activity to that of the deposited bacterial strain of this case when the bacterial strain is tested by the confirmation test explained above

Furthermore, one having excellent characteristics of fermentation property and/or one having fermentation property in a temperature band for general lactic acid bacterium fermentation are preferable.

MCC2430 of the invention can be proliferated easily, for example, by culturing the bacterial strain. The culture condition and the culture medium are as explained above.

MCC2430 of the invention can be provided as a biologically pure culture.

### <Use of the Helveticus Strain>

The bacterium of the invention and MCC2430 of the invention explained above exhibit a probiotic effect through administration to a subject. For the convenience of explanation, the bacterium of the invention and MCC2430 of the invention are together called "the *helveticus* strain" below.

The *helveticus* strain can be used in the form of the bacterium itself, a culture or processed bacterial cell material. That is, after culturing, the culture obtained may be used directly, or a diluted or concentrated culture or bacterial cells collected from the culture may also be used. Moreover, the *helveticus* strain may be the viable bacterium or the dead bacterium or may contain both the viable bacterium and the dead bacterium. Furthermore, processed bacterial cell material of the *helveticus* strain may also be used.

The dead bacterium may be dead bacterium sterilized by heating, lyophilization or the like. Other preparation methods of dead bacterial cells include spray drying method, retort sterilization method, lyophilization method, UHT pasteurization method, pressurized sterilization method, high-pressure steam sterilization method, dry heat sterilization method, flow steam sterilization method, electromagnetic wave sterilization method, electron beam sterilization method, high-frequency sterilization method, radiation sterilization method, ultraviolet sterilization method, ethylene oxide gas sterilization method, hydrogen peroxide gas plasma sterilization method, chemical sterilization method (alcohol sterilization method, formalin fixation method or electrolyzed water treatment method) and the like.

Examples of the processed bacterial cell material include fixed bacterial cells obtained by fixing bacterial cells with acrylamide, carrageenan or the like, a disrupted cell preparation in which the cell walls and the cell membranes of the bacterial cells of the *helveticus* strain are partially or completely disrupted, a centrifugation supernatant thereof (a water-soluble fraction), a fraction obtained by partially purifying the supernatant by ammonium sulfate treatment or the like, and a concentrate of the supernatant. The disrupted cell preparation may be obtained by disrupting the viable bacterium or disrupting the dead bacterium or may be one subjected to heating, lyophilization or the like after disruption.

Moreover, as the disruption, for example, physical disruption, enzymatic lysis treatment, chemical treatment, or autolysate treatment using a method and a device known in the technical field can be selected.

The physical disruption may be by treatment in the state of a bacterial cell suspension or treatment in the state of a bacterial cell powder. As examples of the physical disruption, disruption by stirring using an ultrasonic homogenizer, homogenizer, a ball mill, a bead mill, a Dyno-mill, a planetary mill or the like, disruption by pressure using a jet mill, a french press, cell disruptor or the like or disruption by damaging the bacterial cells through filtration treatment with a filter can be selected.

As the enzymatic lysis treatment, for example, the cell structure of the bacterial cells can be broken using an enzyme such as lysozyme.

As the chemical treatment, the cell structure of the bacterial cells can be broken using a surfactant such as soybean phospholipids and glycerin fatty acid esters.

As the autolysate treatment, the bacterial cells can be lysed with some enzymes of the lactic acid bacterium itself.

At least one kind selected from the *helveticus* strain, a culture of the *helveticus* strain and processed bacterial cells of the *helveticus* strain can be used as an active ingredient. For the convenience of explanation, "at least one kind selected from the *helveticus* strain, a culture of the *helveticus* strain and processed bacterial cell material of the *helveticus* strain" is sometimes referred to as the "the component" below.

The component may be used for a human or a nonhuman animal (preferably a mammal) and is preferably used for a human, a pet and a domestic animal, and more preferably used for a human.

Furthermore, the subject to which the component is applied is not particularly limited as long as the subject needs a probiotic effect, and examples thereof include an infant, a small child, a child, an adult, a healthy individual, a middle-aged individual, an aged individual, and those with poor enteric environment. Of these, the present technique is preferably used for an infant, a small child, an adult or an aged individual.

Moreover, the component has fewer side effects and is highly safe and thus can also be taken continuously for a long time.

The component can also be used effectively for a symptom or a disease which can be prevented, improved or treated with probiotics.

The component has an activity of inducing the production of type I IFN by activating pDCs and/or mDCs in the body of the subject of application.

Thus, the component can be used for inducing the production of type I IFN. More specifically, the component can be used for inducing the production of IFNα and/or IFNβ. Here, the use is not limited to use for a therapeutic purpose and may be use for a non-therapeutic purpose.

In other words, the invention also relates to a method for inducing the production of type I IFN including administering the component to a subject of application. More specifically, the invention also relates to a method for inducing the production of IFNα and/or IFNβ including administering the component to a subject in need of induction of type I IFN production. Moreover, the invention also relates to a non-therapeutic administration method including administering the component to a subject in need of induction of type I IFN production.

In addition, the component can be used for producing an agent for inducing the production of type I IFN.

The subject in need of induction of type I IFN production is not particularly limited as long as the subject needs type I IFN production-inducing effect, and examples thereof include a subject in need of prevention or treatment of viral infection, a subject in need of immunostimulation, a subject in need of treatment or prevention of an immune disease, a subject in need of treatment or prevention of a cancer, a subject in need of treatment or prevention of inflammation, and a subject in need of prevention of a decrease in bone strength.

Type I IFN whose production is induced by the component has (1) a function of increasing the resistance of cells to a virus by suppressing viral replication, (2) a function of increasing the expression of MHC class I molecules in cells which are not infected with a virus and protecting against attacks of NK cells and (3) a function of activating NK cells and eliminating virally infected cells.

That is, the component having an activity of inducing the production of type I IFN can be used for preventing or treating viral infection. Here, the use is not limited to use for a therapeutic purpose and may be use for a non-therapeutic purpose. In other words, the invention also relates to a method for preventing or treating viral infection including administering the component to a subject in need of prevention or treatment of viral infection. Moreover, the invention also relates to a non-therapeutic administration method including administering the component to a subject in need of prevention or treatment of viral infection.

In addition, the component can be used for producing an agent for preventing or treating viral infection.

The prophylactic or therapeutic effect of the component on viral infection can be observed, for example, when the incidence rate of the viral infection is decreased through intake of a composition containing the component, but the confirmation method is not limited thereto.

The subject in need of prevention or treatment of viral infection is not particularly limited as long as the subject needs an effect of preventing or treating viral infection, and an example thereof is a subject infected with a virus.

Type I IFNs are involved in the expression of functions of macrophages and T cells and have an activity of promoting the activation of NK cells, neutrophils and the like. Type I IFNs play an important role in the innate immune system, and an immunostimulating effect is obtained when the production thereof is induced.

That is, the component having an activity of inducing the production of type I IFN can be used for immunostimulation. Here, the use is not limited to use for a therapeutic purpose and may be use for a non-therapeutic purpose. In other words, the invention also relates to an immunostimulating method including administering the component to a subject in need of immunostimulation. Moreover, the invention also relates to a non-therapeutic administration method including administering the component to a subject in need of immunostimulation.

In addition, the component can be used for producing an immunostimulant.

The immunostimulating effect of the component can be observed, for example, when NK cells or macrophages in the peripheral blood are activated through intake of a composition containing the component, but the confirmation method is not limited thereto.

The subject in need of immunostimulation is not particularly limited as long as the subject needs an immunostimulating effect, and an example thereof is a subject with a decrease in the functions of immune cells due to aging or the like.

Type I IFNs have been found to be effective also for treating an autoimmune disease such as multiple sclerosis and are also clinically applied. Moreover, therapeutic effects on various autoimmune diseases such as colitis, encephalomyelitis, arthritis and neonatal inflammation have also been observed.

That is, the component having an activity of inducing the production of type I IFN can be used for treating or preventing an immune disease. Here, the use is not limited to use for a therapeutic purpose and may be use for a non-therapeutic purpose.

In other words, the invention also relates to a method for treating or preventing an immune disease including administering the component to a subject in need of treatment or prevention of an immune disease. Moreover, the invention also relates to a non-therapeutic administration method including administering the component to a subject in need of treatment or prevention of an immune disease.

In addition, the component can be used for producing an agent for treating or preventing an immune disease.

The therapeutic or prophylactic effect of the component on an immune disease can be observed, for example, when the activation of T cells in the peripheral blood is suppressed through intake of a composition containing the component, but the confirmation method is not limited thereto.

The subject in need of treatment or prevention of an immune disease is not particularly limited as long as the subject needs an effect of treating or preventing an immune disease, and an example thereof is a subject with a high autoantibody level in the peripheral blood.

Type I IFNs have activities of suppressing cell proliferation and activating NK cells and the like, and thus type I IFNs have been found to have an anticancer activity through an indirect activity through immunoreaction or a direct activity on tumor cells and are also clinically applied.

That is, the component having an activity of inducing the production of type I IFN can be used for treating or preventing a cancer. Here, the use is not limited to use for a therapeutic purpose and may be use for a non-therapeutic purpose.

In other words, the invention also relates to a method for treating or preventing a cancer including administering the component to a subject in need of treatment or prevention of a cancer. Moreover, the invention also relates to a non-therapeutic administration method including administering the component to a subject in need of treatment or prevention of a cancer.

In addition, the component can be used for producing an agent for treating or preventing a cancer.

The therapeutic or prophylactic effect of the component on a cancer can be observed, for example, when NK cells in the peripheral blood are activated or when the progression-free survival period (the period between the start of treatment and progress of the cancer or the death of the subject) is extended through intake of a composition containing the component, but the confirmation method is not limited thereto.

The subject in need of treatment or prevention of a cancer is not particularly limited as long as the subject needs an effect of treating or preventing a cancer, and an example thereof is a subject having renal cell carcinoma.

Type I IFNs mediate inhibition of the products of proinflammatory genes through the ability of inducing interleukin 10 (IL-10), which is an immunosuppressive cytokine. Moreover, type I IFNs also induce other immunosuppressive mediators such as suppressor of cytokine signaling 1 (SOCS-1) and tristetraprolin (TTP), which act on homeostasis restoration in the immune system through a variety of mechanisms.

That is, the component having an activity of inducing the production of type I IFN can be used for treating or preventing inflammation. Here, the use is not limited to use for a therapeutic purpose and may be use for a non-therapeutic purpose.

In other words, the invention also relates to a method for treating or preventing inflammation including administering the component to a subject in need of treatment or prevention of inflammation. Moreover, the invention also relates to a non-therapeutic administration method including administering the component to a subject in need of treatment or prevention of inflammation.

In addition, the component can be used for producing an anti-inflammatory agent.

The therapeutic or prophylactic effect of the component on inflammation can be observed, for example, when the proportion of regulatory T cells or the production of IL-10 in the peripheral blood is increased through intake of a composition containing the component, but the confirmation method is not limited thereto.

The subject in need of treatment or prevention of inflammation is not particularly limited as long as the subject needs an effect of treating or preventing inflammation, and an example thereof is a subject with a high risk of chronic inflammation due to aging, obesity or the like.

Osteoclasts play a role responsible for metabolism of bones, but an excessive increase thereof decreases bone strength. It has been found that type I IFNs have an activity of suppressing formation of osteoclasts and contribute to prevention of a decrease in bone strength.

That is, the component having an activity of inducing the production of type I IFN can be used for preventing a decrease in bone strength. Here, the use is not limited to use for a therapeutic purpose and may be use for a non-therapeutic purpose.

In other words, the invention also relates to a method for preventing a decrease in bone strength including administering the component to a subject in need of prevention of a decrease in bone strength. Moreover, the invention also relates to a non-therapeutic administration method including administering the component to a subject in need of prevention of a decrease in bone strength.

In addition, the component can be used for producing an agent for preventing a decrease in bone strength.

The prophylactic effect of the component on a decrease in bone strength can be observed, for example, when a decrease in the bone density is suppressed through intake of a composition containing the component, but the confirmation method is not limited thereto.

The subject in need of prevention of a decrease in bone strength is not particularly limited as long as the subject needs an effect of preventing a decrease in bone strength, and an example thereof is a subject who is 50 years old or older and whose bone strength is decreasing.

Regarding the administration or the intake of the component, the component is preferably taken for at least one week, more preferably taken continuously for at least four weeks, and desirably taken every day.

The used amount of the component is not particularly restricted because of the high safety, but for example, the amount is preferably 1×10⁶ to 1×10¹² cfu/kg body weight/day, more preferably 1×10⁷ to 1×10¹¹ cfu/kg body weight/day, further preferably 1×10⁸ to 1×10¹⁰ cfu/kg body weight/day.

The used amount (dose) per one individual (body weight) is preferably 10⁷ to 10¹⁴ cfu/day, more preferably 10⁸ to 10¹³ cfu/day, further preferably 10⁹ to 10¹² cfu/day.

Moreover, the used amount of the component is preferably 0.01 to 100 mL/body weight kg/day, more preferably 0.1 to 10 mL/body weight kg/day.

In the present specification, the cfu means colony forming unit and indicates colony forming unit. When the bacterium is a dead bacterium, the cfu can be replaced with the cells.

### <Composition>

The component can be used as it is. Moreover, the component can also be used after mixing with a general carrier or diluent or the like which is acceptable physiologically or pharmaceutically or in terms of foods and drinks. That is, the invention also relates to a composition containing at least one kind selected from the bacterium of the invention, a culture of the bacterium, processed bacterial cell material of the bacterium, MCC2430 of the invention, a culture of the bacterial strain and processed bacterial cells of the bacterial strain. For the convenience of explanation, the above composition is referred to as the composition of the invention below.

The component contained in the composition of the invention exhibits various pharmacological effects explained above. That is, the composition of the invention can be provided as a composition for inducing type I IFN production. Moreover, the composition of the invention can be provided as at least one kind selected from the group consisting of an antiviral composition, an immunostimulating composition, a composition for treating or preventing an immune disease, an anticancer composition, an anti-inflammatory composition and a composition for preventing a decrease in bone strength.

The subject of administration of the composition of the invention is not particularly limited but may be any of the various subjects explained above. That is, the composition of the invention can be provided, for example, to a subject who needs type I IFN production-inducing effect. The composition of the invention can also be provided, for example, to a subject who needs an effect of preventing or treating viral infection, a subject who needs an immunostimulating effect, a subject who needs an effect of treating or preventing an immune disease, a subject who needs an effect of treating or preventing a cancer, a subject who needs an effect of treating or preventing inflammation, and a subject who needs an effect of preventing a decrease in bone strength.

The component can also be contained in the composition as an active ingredient and can also be used for products for a wide variety of uses such as pharmaceutical products and foods and drinks because of the high safety. Such products can be produced appropriately using optional components suitable for the respective uses by known production methods suitable for the respective uses.

In the present specification, the composition has the meanings including a food or drink composition, a pharmaceutical composition, a feed and the like.

Embodiments for providing the composition of the invention as a pharmaceutical product and providing as a food or a drink are described in detail below.

### <Pharmaceutical Composition>

The composition of the invention can be provided as a pharmaceutical composition. The pharmaceutical composition of the invention is not particularly limited as long as the pharmaceutical composition contains the component.

The pharmaceutical composition of the invention may be in the form using the component directly or the form formulated by blending a physiologically acceptable liquid or solid carrier for formulation.

The pharmaceutical composition of the invention can be safely administered also to a patient having various diseases. It is expected that the invention does not easily cause side effects even with continuous administration for a long time. Moreover, the invention can be administered safely also to an infant, a small child or a child. Thus, the invention is also preferable for preventing, improving and/or treating a disease or a symptom thereof of an infant, a small child or a child.

The invention may be use for a therapeutic purpose or use for a non-therapeutic purpose.

The "non-therapeutic purpose" is a concept which does not include medical practice, namely treatment of a human body by therapy. Examples thereof include health promotion and beauty treatment.

The "improvement" means: improvement of a disease, a symptom or the condition; prevention or delay of deterioration of a disease, a symptom or the condition; or reversal, prevention or delay of progress of a disease or a symptom.

The "prevention" means prevention or delay of the onset of a disease or a symptom in the subject of the application or reduction of the risk of a disease or a symptom of the subject of the application.

The dosage form of the pharmaceutical composition of the invention is not particularly restricted, and specifically, examples thereof include tablets, pills, powder, liquid preparation, suspensions, emulsions, granules, capsules, syrups, suppositories, injections, ointment, patches, eye drops, and nasal drops. Moreover, for the formulation, an additive which is generally used as a carrier for formulation, such as excipients, binders, disintegrating agents, lubricants, stabilizers, flavoring agents, diluents, surfactants and solvents for injection, can be used.

For the formulation, a component which is generally used for formulation, such as excipients, pH-adjusting agents, colorants and corrigents, can be used for the pharmaceutical composition according to the invention. As long as the effects of the invention are not impaired, a component having an effect of preventing, improving and/or treating a disease or a symptom which is known or will be found in the future and which is relevant to the invention can also be used for the pharmaceutical composition according to the invention.

In addition, the formulation can be appropriately conducted by a known method depending on the dosage form. The formulation may also be conducted by appropriately blending a carrier for formulation.

The amount of the *helveticus* strain contained in the pharmaceutical composition of the invention is appropriately determined based on the dosage form, the usage, the age of the patient, the gender, the type of disease, the degree of the disease, the other conditions and the like, but is generally preferably in the range of 1×10⁶ to 1×10¹² cfu/g or 1×10⁶ to 1×10¹² cfu/mL, more preferably in the range of 1×10⁷ to 1×10¹¹ cfu/g or 1×10⁷ to 1×10¹¹ cfu/mL. When the *helveticus* strain is a dead bacterium, cfu/g or cfu/mL can be replaced with cells/g or cells/mL.

The timing of administration of the pharmaceutical composition of the invention is not particularly limited, and the timing of administration can be appropriately chosen according to the method for treating the subject symptom or disease. The pharmaceutical composition may be administered prophylactically or used for a maintenance therapy. Moreover, the form of administration is preferably determined based on the formulation form, the age of the patient, the gender, the other conditions, the degree of the symptom of the patient or the like. In this regard, in all the cases, the pharmaceutical composition of the present technique can be administered once a day or in separate portions and may be administered once in several days or in several weeks.

### <Food or Drink Composition>

Furthermore, the composition of the invention can be used as a food or drink composition. The food or drink composition of the invention may be produced by adding the component to a known food or a known drink, or a new food or drink composition can be produced by mixing the component into a raw material of a food or a drink.

The food or drink composition of the invention is not particularly limited as long as the component is contained, and examples of the food or drink composition include: drinks such as soft drinks, carbonated drinks, nutritional drinks, fruit juices and lactic acid bacteria beverages (including concentrated undiluted solutions of the drinks and powders for preparation thereof); frozen desserts such as ice creams, sherbets and shaved ice; confectioneries such as sweets, chewing gums, candies, gums, chocolates, tablet candies, snacks, biscuits, jellies, jams, creams and baked sweets; dairy products such as processed milk, milk beverages, fermented milk, drink yogurts and butter; breads; liquid foods such as enteral nutrition products and porridge, baby foods, infant formula or sport drinks; and other functional foods. Moreover, the food or the drink may be a supplement and may be, for example, a supplement in the tablet form. In the case of a supplement, the *helveticus* strain can be taken while the amount of meals and the calorie intake per day are not affected by other foods.

The food or drink composition of the invention may contain various components as long as the effects of the invention are not impaired. For example, the food or drink composition of the invention may contain the following components: proteins such as whey protein, casein protein, soybean protein and pea protein or mixtures or decomposition products thereof; amino acids such as leucine, valine, isoleucine and glutamine; saccharides such as galactose, glucose, fructose, mannose, cellobiose, maltose, lactose, sucrose, trehalose, dextrin and starch and human milk oligosaccharides such as 2'-fucosyllactose, 3'-fucosyllactose, lacto-N-fucopentaose I, lacto-N-difucohexaose I, lacto-N-tetraose and lacto-N-neotetraose; vitamins such as vitamin B6 and vitamin C; creatine; citric acid; or fish oil.

The food or drink composition of the invention can be sold as a food or drink composition or a food or a drink with a label with use for inducing type I IFN production, suppressing viral infection, antiviral use, immunostimulation, treating or preventing an immune disease, anticancer use, antiinflammation or preventing a decrease in bone strength. Moreover, the food or drink composition of the invention can be sold as a food or drink composition or a food or a drink with a label with use for preventing or treating a disease or a symptom which can be prevented or treated through induction of type I IFN production. The food or drink composition or the food or the drink of the invention can also be labeled with "those who want to suppress viral infection", "those who want to maintain immune function", "act on plasmacytoid dendritic cells and help maintaining immune function of healthy individuals", "those who want to suppress an allergy", "relieve discomfort in the nose caused by allergens", "relieve discomfort in the eyes caused by allergens", "those who want to suppress inflammation", "those who want to maintain bone components", "those who want to maintain strong bones", "help bone health through a function of maintaining bone components" or the like. In addition, of course, a term can be used as long as the term indicates a secondary effect caused by inducing type I IFN production.

The food or drink composition of the invention can also be provided or sold as a food or drink composition or a food or a drink with a label with use of probiotics or the like (including a health use). Moreover, the food or drink composition or the food or the drink can be provided or sold with a label for "those who need to lead a life with *Lactobacillus* spp.", "those who need to lead a life with lactic acid bacteria", "those who want to improve the enteric environment", "those who want to correct the stomach condition", "those who want to form a good enteric environment", "improve the enteric environment" or the like as the subject of intake.

The "label" means all the acts for informing a consumer of the use, and all the labels which remind of or cause to guess the use are the "labels" of the invention, regardless of the purposes of the labels, the contents of the labels, the objects to be labeled, the media and the like. However, labeling with an expression which allows a consumer to directly recognize the use is preferable.

Specifically, examples include an act of describing the use on a product regarding the food or drink composition or the food or the drink of the invention or on packaging of a product, an act of transferring an article in which the use is described on a product or packaging of a product, delivering such an article, displaying such an article for transfer or delivery, or importing such an article, an act of displaying or distributing an advertisement of a product, a price list or a business document with a description of the use thereon, or providing information with such contents with a description of the use by an electromagnetic method (the Internet or the like) and other acts, and in particular, labeling on packaging, a container, a catalogue, a brochure, an advertisement material in a sales site such as POP, other documents or the like is preferable.

The label is preferably a label approved by the administration or the like (for example, a label approved based on various systems provided by the administration and provided in the form based on the approval). Examples include labels with food with health claims, more specifically food with health claims, health foods, functional foods, enteral nutrition products, food for special dietary uses, foods with nutrient function claims, or quasi-drugs, and other examples include labels approved by the Consumer Affairs Agency, such as foods for specified health uses, foods with nutrient function claims and foods with function claims, and labels approved by a similar system. Examples of the latter include a label with foods for specified health uses, a label with qualified foods for specified health uses, a label indicating influence on the structure or the function of a body, a label with reduction of disease risk, and a label with a scientifically grounded function. More specifically, examples include labels with food for specified health uses (especially labels with health uses) provided by the Cabinet Office Ordinance on Labeling Permission for Special Dietary Uses under the Health Promotion Act (Cabinet Office Ordinance No. 57 on August 31, 2009), and similar labels.

The amount of the *helveticus* strain contained in the food or drink composition of the invention is appropriately determined based on the form of the food or drink composition, and the amount in the food or the drink is generally preferably in the range of 1×10⁶ to 1×10¹² cfu/g or 1×10⁶ to 1×10¹² cfu/mL, more preferably in the range of 1×10⁷ to 1×10¹¹ cfu/g or 1×10⁷ to 1×10¹¹ cfu/mL.

An example of the food or drink composition according to the invention is a powdered formula for infants and small children (also called infant powdered formula). The infant powdered formula refers to a powdered formula for infants and small children for infants of the age of 0 to 12 months, follow-up milk for infants and younger children of the age of 6 to 9 months or older (until the age of 3 years), a powdered formula for low birthweight infants for newborns with a body weight at birth less than 2500 g (low birthweight infants), a formula for treatment used for treating infants and small children with sickness such as milk allergy and lactose intolerance, and the like. The composition can also be applied to food with health claims or food for the ill. The system for food with health claims has been established not only for general foods but also for foods in the form of tablets, capsules and the like, in view of the trends inside and outside of Japan and also considering the consistency with the existing system for foods for specified health uses. The system includes two types, namely foods for specified health uses (individual approval system) and foods with nutrient function claims (standard regulation system).

In the invention, the form of the formula for infants and small children can be the form of a liquid formula in addition to a powdered formula.

### <Production Method>

The method for producing the composition of the invention is explained below. The method for producing the composition of the invention has a step of adding the component to a raw material. In the invention, the step of adding the component may be conducted at any stage in the production step.

Moreover, the method may have a step of adding the *helveticus* strain to a raw material and fermenting the raw material. In this case, the composition of the invention can be provided as a fermented composition.

Examples of the method for producing the composition of the invention are shown below, but the method is not limited thereto.

An example of the production method is a method for producing a composition including at least one of the step (a) below and the step (b) below.
(a) A step of mixing the component and a prebiotic
(b) A step of mixing the component and a milk component

Here, a composition containing the component may be used as the component. Furthermore, the composition may further contain a probiotic such as other *Lactobacillus* strains, other lactic acid bacteria and *Bifidobacterium* strains.

The step of mixing the prebiotic and/or the milk component may be at any stage in the production step. For example, when the milk component is mixed, the step may be at the same time as the step (a), before the step (a) or after the step (a).

Moreover, the milk component is preferably a powder and is more preferably mixed with a bacterial powder of the *helveticus* strain.

Another example of the method for producing the composition of the invention is a method for producing a composition including the steps (A) and (B) below.
(A) A step of culturing the *helveticus* strain in a culture medium and thus obtaining a culture or a product containing the *helveticus* strain; and
(B) a step of drying the culture or the product and thus obtaining a bacterial powder or a dried product:
   can be provided. The drying is preferably lyophilization or spray drying.

The milk component is not particularly limited, and examples thereof include cow's milk, buffalo's milk, sheep's milk, goat's milk, mare milk, skim milk, concentrated skim milk, skim milk powder, concentrated milk, whole milk powder, cream, butter, buttermilk, condensed milk, a milk oligosaccharide, and a milk protein. One kind or two or more kinds selected from the group consisting thereof can be used. The milk protein is not particularly limited, and examples thereof include whey, casein, and hydrolysates thereof. One kind or two or more kinds selected from the group consisting thereof can be used. Of the milk components, a component derived from cow's milk is preferable.

The milk oligosaccharide is not limited to a cow milk oligosaccharide and may be, for example, a human milk oligosaccharide. The human milk oligosaccharide is not particularly limited, and examples thereof include 2'-fucosyllactose, 3'-fucosyllactose, lacto-N-fucopentaose I, lacto-N-difucohexaose I, lacto-N-tetraose, and lacto-N-neotetraose. One kind or two or more kinds selected from the group consisting thereof can be used.

Regarding the hydrolysates, a whey hydrolysate, a casein hydrolysate or the like can be produced by hydrolyzing the milk component (preferably a milk protein). Examples of the hydrolysis include acid and alkali hydrolysis and enzymatic hydrolysis. Of these, enzymatic hydrolysis is preferable, and a proteolytic enzyme is preferable as the enzyme. Examples of the proteolytic enzymes include protease, tripepsin, chymotrypsin, plasmin, pepsin, papain, peptidase, and aminopeptidase. The pH during the hydrolysis reaction is appropriately adjusted to the optimum pH of the enzyme used, and the hydrolysis reaction can be conducted, for example, at pH 2 to 6. The temperature during the hydrolysis reaction is not particularly limited, and is generally preferably in the range of 30 to 60°C, and the reaction period is preferably two to 10 hours.

The form of the *helveticus* strain or the culture containing the same is not particularly limited and may be liquid or solid, but the form is preferably a dried form (for example, a bacterial powder or a dried culture) in view of easiness of handling during the production and during the storage. Moreover, the culture may be, as described above, bacterial cells themselves from which the culture solution has been separated, a culture containing bacterial cells, a culture from which bacterial cells have been removed or the like.

The drying method is not particularly limited, and may be spray drying method, retort sterilization method, lyophilization method, UHT pasteurization method, pressurized sterilization method, high-pressure steam sterilization method, dry heat sterilization method, flow steam sterilization method, electromagnetic wave sterilization method, electron beam sterilization method, high-frequency sterilization method, radiation sterilization method, ultraviolet sterilization method, ethylene oxide gas sterilization method, hydrogen peroxide gas plasma sterilization method, chemical sterilization method (alcohol sterilization method, formalin fixation method or electrolyzed water treatment method) or the like.

The bacterial cells may be disrupted, and the disrupted may be bacterial cells obtained by disrupting a viable bacterium, bacterial cells obtained by disrupting a dead bacterium, or bacterial cells subjected to heating, lyophilization or the like after disruption.

Of these, the culture is preferably subjected to lyophilization to increase the viability, and the culture is preferably subjected to spray drying to increase the production efficiency.

When a supplement or tablets are produced as the composition of the invention, a step of mixing a culture containing the *helveticus* strain and an excipient or the like and thus obtaining a mixture and a step of forming into the predetermined shape can be included. The forming step is, for example, tableting, and an example of tableting is obtaining tablets by compression molding of a powder or a granulated mixture.

An example of the production method of the invention is a method for producing a fermented food or drink (preferably a fermented milk) containing the *helveticus* strain.

A step of adding the bacterial powder obtained by the production method described above to a raw fermented milk material and obtaining a fermented milk using the *helveticus* strain is conducted. Alternatively, a step of mixing the bacterial powder and a fermented milk and thus obtaining a fermented milk containing the *helveticus* strain is conducted. Regarding the bacterial cell amount of the *helveticus* strain in the composition, the fermentation condition and the blending amount are adjusted to achieve a predetermined bacterial cell amount. As a result, a fermented food or drink (preferably a fermented milk or a fermented product) containing the *helveticus* strain can be provided.

### Examples

The invention is explained in further detail below based on Examples. In this regard, the Examples explained below show examples of typical Examples of the invention, and the scope of the invention is not construed as being narrower due to the Examples.

### (1) Preparation of Heat Sterilized Cells of Lactic Acid Bacterium Strains

The four lactic acid bacterium strains shown in Table 1 were cultured statically in MRS culture medium or M17 culture medium at 30°C or 37°C for 16 hours. After culturing, the cells were collected, washed twice with sterile water and then sterilized by autoclaving under the condition at 90°C for 15 minutes. Then, the bacterial strains were adjusted with sterile water to a concentration of 1x10⁸ cells/mL or 1x10⁹ cells/mL, and thus heat sterilized cells used for the test were obtained.

### [Table 1]

**[Table 1]**

| Bacterial Species | Bacterial Strain Name |
|---|---|
| *Lactobacillus helveticus* | MCC2430 |
| *Lactobacillus helveticus* | SBT2171 |
| *Lactobacillus helveticus* | JCM1120 (type strain) |
| *Lactobacillus lactis* subsp. *Lactis* | JCM5805 |

### (2) Preparation of Dendritic Cells

The dendritic cells (DCs) used for the test were isolated from human peripheral blood mononuclear cells (PBMCs) purchased from STEMCELL Technologies by negative selection using EasySep Human Pan-DC Pre-Enrichment Kit (STEMCELL Technologies). The dendritic cells were further suspended in RPMI-1640 culture medium containing an antibiotic and heat inactivated fetal bovine serum (FBS) having a final concentration of 10% and adjusted to 1x10⁶ cells/mL. In the prepared suspension, plasmacytoid dendritic cells (pDCs) and myeloid dendritic cells (mDCs) coexisted.

### (3) Measurement of Type I IFN Production Amounts of Dendritic Cells by Stimulation of Sterilized Cells

The sterilized cells of the bacterial strains prepared in (1) were added to the suspension of the dendritic cells prepared in (2), and the dendritic cells were cultured in a CO₂ incubator under the condition at 37°C and 5% CO₂.

The sterilized cells of the four bacterial strains above were added in an amount 10 times higher than that of the dendritic cells in terms of the cell count ratio. Moreover, for JCM5805, a test in which 100 times the amount of the dendritic cells in terms of the cell count ratio were added was conducted additionally.

The culture supernatants were collected 24 hours after starting culturing. The concentrations of IFNα were measured by flow cytometry using an IFNα assay kit (Cytometric Bead Array Kit, Becton Dickinson), and the concentrations of IFNβ were measured by ELISA using an IFNβ assay kit (Human IFN-beta DuoSet, manufactured by R&D systems).

### (4) Results

The measurement results of the production amounts of INFα and INFβ in the dendritic cells with stimulation with the sterilized cells of the lactic acid bacterium strains are shown in FIG. 1 and FIG. 2.

*Lactobacillus helveticus* MCC2430 had the highest ability of inducing the production of both INFα and INFβ. Here, it is known that *Lactobacillus helveticus* SBT2171 contributes to an increase in pDCs as described in JP6705628B (Patent Literature 2), and it is known that *Lactobacillus lactis* subsp. *lactis* JCM5805 activates pDCs and promotes IFNα production as described in JP6170190B (Patent Literature 1). It was shown that MCC2430 has a significantly higher type I IFN production-inducing ability than those of the known bacterial strains.

### [Production Examples]

Production Examples of the pharmaceutical composition and the food or drink composition of the invention are shown below, but the composition of the present technique is not limited thereto.

### [Production Example 1]

*Lactobacillus helveticus* MCC2430 is added to an MRS liquid culture medium and anaerobically cultured at 37°C for 16 hours, and then the culture solution is concentrated and lyophilized to obtain a lyophilized powder of the bacterium (a bacterial powder). The bacterial powder and whey protein concentrate (WPC) are mixed uniformly, and thus a composition is obtained. A composition containing *Lactobacillus helveticus* MCC2430 of the invention is obtained by dissolving 20 g of the composition in 200 g of water.

### [Production Example 2]

*Lactobacillus helveticus* MCC2430 is added to an MRS liquid culture medium and anaerobically cultured at 37°C for 16 hours, and then the bacterial cells are washed three times by centrifuging and suspending again in distilled water, then suspended in distilled water at 10 mg (in terms of bacterial cell weight)/ml and heat sterilized at 100°C for 15 minutes. Thus, a solution containing the heat sterilized cells is obtained. The solution containing the heat sterilized cells is concentrated and lyophilized to obtain a lyophilized powder of the bacterium (a bacterial powder). The bacterial powder and an oligosaccharide are mixed uniformly, and thus a composition is obtained. As the oligosaccharide, human milk oligosaccharides such as 2'-fucosyllactose, 3'-fucosyllactose, lacto-N-fucopentaose I, lacto-N-difucohexaose I, lacto-N-tetraose and lacto-N-neotetraose, isomaltooligosaccharides, lactulose, raffinose, fructooligosaccharides, galactooligosaccharides and soybean oligosaccharides can be used.

### [Production Example 3]

*Lactobacillus helveticus* MCC2430 is added to an MRS liquid culture medium and cultured at 37°C for 16 hours, and then the culture solution is concentrated and lyophilized to obtain a lyophilized powder of the bacterium (a bacterial powder). The bacterial powder and a dry powder of milk protein concentrate (MPC480, manufactured by Fonterra Co-operative Group Limited, protein content of 80 mass%, casein protein:whey protein = about 8:2) are mixed uniformly, and thus a composition is obtained. A composition containing *Lactobacillus helveticus* MCC2430 of the invention is obtained by dissolving 20 g of the composition in 200 g of water.

### [Production Example 4]

*Lactobacillus helveticus* MCC2430 is added to an MRS liquid culture medium and cultured at 37°C for 16 hours, and then the culture solution is concentrated and lyophilized to obtain a lyophilized powder of the bacterium (a bacterial powder) containing *Lactobacillus helveticus* MCC2430 of the invention. Next, the bacterial powder and crystalline cellulose are introduced into an agitation granulator and mixed. Then, purified water is added for granulation, and the granules are dried to obtain granules (a pharmaceutical composition) containing a fermented component of the bacterium and the excipient. In this manner, granules containing *Lactobacillus helveticus* MCC2430 of the invention can be obtained.

### [Production Example 5]

A production method of a fermented milk containing *Lactobacillus helveticus* MCC2430 is shown below.

First, a raw milk material, water according to the need, other components and the like are mixed, preferably homogenized and heat sterilized. The homogenization and the heat sterilization can be conducted by general methods. A lactic acid bacterium starter is added (seeded) to the heat sterilized prepared milk solution, and the solution is kept at a predetermined fermentation temperature and fermented to obtain a fermented product. Through fermentation, curd is formed.

As the lactic acid bacterium starter, for example, lactic acid bacteria which are generally used for yogurt production, such as *Lactobacillus bulgaricus, Lactococcus lactis* and *Streptococcus thermophilus,* can be used. When the pH reaches the target value, the formed curd is crushed by stirring and cooled to 10°C or lower, and a fermented product is thus obtained. By cooling to 10°C or lower, the activity of the lactic acid bacterium can be reduced, and the production of an acid can be suppressed.

Next, the fermented product obtained by the fermentation step is heat treated, and a fermented product after the heating (a fermented product after the heat treatment) is thus obtained. By appropriately heating the fermented product, the production of an acid by the lactic acid bacterium in the fermented product after the heating can be inhibited. As a result, a decrease in pH during the subsequent production step and/or during the storage can be inhibited.

Next, *Lactobacillus helveticus* MCC2430 is added to the fermented product after the heating obtained by the heat treatment step. The amount of *Lactobacillus helveticus* MCC2430 added, relative to the fermented product after the heating, is preferably 1×10⁷ to 1×10¹¹ cfu/mL, more preferably 1×10⁸ to 1×10¹⁰ cfu/mL. When the *Lactobacillus helveticus* MCC2430 is a dead bacterium, cfu/mL can be replaced with cells/mL.

After adding *Lactobacillus helveticus* MCC2430 to the fermented product after the heating, the mixture is concentrated. The concentration step can be conducted appropriately using a known concentration method. For example, centrifugation method or membrane separation method can be used.

Through the centrifugation method, the whey in the product to be concentrated is removed, and a concentrated fermented milk which has an increased solid content concentration is obtained.

A fermented milk containing *Lactobacillus helveticus* MCC2430 of the invention can be produced as described above.

By taking or administering the fermented milk containing *Lactobacillus helveticus* MCC2430 of the invention obtained as described above, type I IFN production-inducing effect, a viral infection-suppressing effect, an antiviral effect, an immunostimulating effect, an effect of treating or preventing an immune disease, an anticancer effect, an anti-inflammatory effect and an effect of preventing a decrease in bone strength can be expected to be obtained.

### [Production Example 6]

A production method of a powdered formula containing *Lactobacillus helveticus* MCC2430 is shown below.

In 300 kg of warm water, 10 kg of desalted cow's milk whey protein powder (manufactured by MILEI GmbH), 6 kg of cow's milk casein powder (manufactured by Fonterra Co-operative Group Limited), 48 kg of lactose (manufactured by MILEI GmbH), 920 g of a mineral mixture (manufactured by Tomita Pharmaceutical Co., Ltd), 32 g of a vitamin mixture (manufactured by Tanabe Seiyaku Co., Ltd.), 500 g of lactulose (manufactured by Morinaga Milk Industry Co., Ltd.), 500 g of raffinose (manufactured by Nippon Beet Sugar Manufacturing Co., Ltd.) and 900 g of galactooligosaccharide syrup (manufactured by Yakult Pharmaceutical Industry Co., Ltd.) are dissolved and further heated and dissolved at 90°C for 10 minutes, and after adding 28 kg of modified fats (manufactured by Taiyo Yushi Corp.), the mixture is homogenized. Then, after sterilization and concentration steps, the mixture is spray dried, and about 95 kg of a powdered formula is thus prepared. To the powdered formula, 100 g of a bacterial cell powder of *Lactobacillus helveticus* MCC2430 (1.8×10¹¹ cfu/g, manufactured by Morinaga Milk Industry Co., Ltd.) dispersed in starch is added, and about 95 kg of a powdered formula containing the *Lactobacillus* strain and oligosaccharides is thus prepared. When the obtained powdered formula is dissolved in water and when a formula solution having a total solid content concentration as a standard formula concentration of 14% (w/v) is obtained, 2.7×10⁹ cfu/100 mL can be obtained as the *Lactobacillus* strain count in the formula solution.

The powdered formula above can further contain a human milk oligosaccharide such as 2'-fucosyllactose, 3'-fucosyllactose, lacto-N-fucopentaose I, lacto-N-difucohexaose I, lacto-N-tetraose and lacto-N-neotetraose and a probiotic such as *Lactobacillus* strains and lactic acid bacteria other than *Lactobacillus helveticus* MCC2430 and *Bifidobacterium* strains.

By taking or administering the powdered formula containing *Lactobacillus helveticus* MCC2430 of the invention obtained as described above, type I IFN production-inducing effect, a viral infection-suppressing effect, an antiviral effect, an immunostimulating effect, an effect of treating or preventing an immune disease, an anticancer effect, an anti-inflammatory effect and an effect of preventing a decrease in bone strength can be expected to be obtained.

### Industrial Applicability

The invention can be applied to a food or a drink and a pharmaceutical product containing a lactic acid bacterium.

## Claims

1. A bacterium classified as *Lactobacillus helveticus* which can activate plasmacytoid dendritic cells (pDCs) and/or a myeloid dendritic cell (mDC) and induce production of type I IFN and which induces the production of type I IFN in an amount 1.5 times or more higher than that of *Lactobacillus helveticus* SBT2171 (accession number: Ferm BP-5445).

2. *Lactobacillus helveticus* MCC2430 (accession number: NITE BP-03882) .

3. A composition comprising at least one kind selected from the bacterium according to claim 1 or 2, a culture of the bacterium and processed bacterial cells of the bacterium.

4. The composition according to claim 3 which is a composition for inducing production of type I IFN.

5. The composition according to claim 3 which is at least one kind selected from the group consisting of an antiviral composition, an immunostimulating composition, a composition for treating or preventing an immune disease, an anticancer composition, an anti-inflammatory composition and a composition for preventing a decrease in bone strength.

6. The composition according to claim 3 which is a food or a drink or a pharmaceutical product.

7. A method for producing a composition comprising a step of adding at least one kind selected from the bacterium according to claim 1 or 2, a culture of the bacterium and processed bacterial cells of the bacterium to a raw material.

8. The production method according to claim 7 comprising a step of fermenting the raw material with the bacterium, wherein the composition is a fermented composition.
